# EUROPEAN PATENT SPECIFICATION

(11) **EP 1 389 136 B1**
(45) Date of publication and mention of the grant of the patent: **05.10.2005**
(21) Application number: 02745161.6
(22) Date of filing: 21.05.2002
(51) Int. Cl.: A61L 28/00, A61F 5/448

(54) **AN OSTOMY APPLIANCE**
OSTOMIE-VORRICHTUNG
ACCESSOIRE POUR STOMIE

(30) Priority: 21.05.2001 DK 200100821
(43) Date of publication of application: 18.02.2004
(73) Proprietor: COLOPLAST A/S, 3050 Humlebaek (DK)
(72) Inventor: HAGEDORN-OLSEN, Lars, DK-3000 Helsingor (DK)
(74) Representative: Nilausen, Kim
(86) International application number: PCT/DK2002/000338
(87) International publication number: WO 2002/094333

(56) References cited:
- EP-A- 0 317 326
- EP-A- 0 415 592
- EP-A- 0 700 777
- WO-A-00/53133
- US-A- 5 607 413

## Description

### BACKGROUND OF THE INVENTION

### 1. Field of the Invention

The present invention relates to an ostomy appliance and more particularly to a convex element and an ostomy appliance comprising such convex element.

In connection with surgery for a number of diseases in the gastro-intestinal or urinary tract a consequence is, in many cases, that the colon, the ileum or the ureter has been exposed surgically and the patient is left with an abdominal stoma, or, in nephrostomy or ureterostomy, the ureter or a catheter is exposed in the back or the chest region or abdominal region, and the effluents or waste products of the body, which are conveyed through these organs, are discharged through the artificial orifice or opening and are collected in a collection bag, which is usually adhered to the skin by means of an adhesive wafer or plate having an inlet opening for accommodating the stoma/ureter/catheter. Also in connection with a fistula, the patient will have to rely on an appliance to collect the bodily material emerging from such opening.

Ostomy appliances are well known. Such appliances may be two-piece or one-piece appliances. In both types of appliances, an adhesive barrier member (or base plate) is attached to the wearer's abdomen/back/chest. In case of a one-piece appliance, a receiving member or bag is attached to the base plate. In case of a two-piece appliance, the adhesive barrier member forms part of a body side member and a receiving member or bag is attached releasably to the body side ostomy member for receiving exudates from the stoma.

When using one-piece appliances, the whole appliance, including the adhesive skin barrier securing the appliance to the skin is normally removed and replaced by a fresh appliance. When using two-piece appliances, the body side ostomy member is left in place up to several days, and only the receiving member or bag attached to the body side member is replaced. The attachment means for attaching an ostomy receiving bag may be a system known per se comprising matching coupling rings or matching flanges and adhesive surfaces engaging with and sealing against a flange area of the body side member.

The production of ostomy products with a flat proximal adhesive skin barrier includes producing a plane disc of adhesive suitable for use on the human skin which is provided with a plastics sheet on both sides. The plastics sheet on the distal side of the adhesive (facing away from the ostomate) is attached, e.g. by gluing or welding, to a collection bag or to a coupling part, to which a collecting bag may be detachably coupled. The sheet on the proximal side of the adhesive is a protective sheet, which is removed prior to use, and the ostomy appliance is then adhered to the user's skin by means of the substantially plane proximal side of the adhesive.

In ostomy patients it is frequently seen that the closest surroundings of the stoma, at a distance of 1-2 cm, are recessed or are positioned in a crater or a cavity as compared to the rest of the skin surface surrounding the stoma. For such patients it has been found to be expedient to use an ostomy product where the adhesive surface around the opening for receiving the stoma has a part which is convex and protrudes toward the user in order to enable the adhesive face of the ostomy appliance to engage and adhere to the skin everywhere in the crater or the cavity. In particular, it is important that the ostomy appliance adheres well to the skin in the area next to the stoma in order to give security against deterioration of the adhesive and against leakage. The use of an adhesive wafer having a rigid convex shape may also apply an external pressure to the area next to the stoma which, in particularly in connection with recessed stomas, will ensure a sufficient protrusion of the stoma to aid the discharge of effluents therefrom directly into the collection bag without contacting the exposed adhesive next to the stoma and prolong the time of service of the adhesive wafer or plate. The shape of the protruding part of the adhesive face may e.g. be domed or conical, and such products are known under the designation convex products. Throughout the specification the term convex has this broad meaning irrespective of the actual embodiment.

### 2. Description of the Related Art

Traditionally such a rigid convex shape may be provided by inserting a relatively rigid convex adapter, e.g. of the type disclosed in WO 93/04646 which is typically made of a material such as EVA or of the type disclosed in WO 93/18725 which is typically made of a rigid or semi-rigid polymer material such as polypropylene, HD polyethylene or polystyrene.

In EP 317326 is disclosed a pressure ring assembly having three essential elements, namely firstly, a relatively rigid support ring, preferably formed of a thermoplastic material, secondly, an intermediate ring of a soft, resilient, moisture-impermeable, thermoformable material which is secured to the convex proximal surface of the rigid support ring and thirdly a barrier ring of soft, deformable moisture-absorbing skin barrier material having both dry and wet tack.

US Patents Nos. 5,607,413 and 5,730,735 disclose convex ostomy faceplates comprising a convex pressure ring having a deep annular recess in order to reduce the weight of the faceplate and render the ring more flexible and conformable in use than it would be if the recess was absent. Furthermore, the inner connection part of the pressure ring is thin and flexible in order to maintain flexibility and so thin as to be easily cut with scissors to enlarge the stoma receiving hole of the faceplate.

Although such convex pressure ring offers a degree of flexibility, it may give rise to problems in the production as the risk of damaging the convex pressure ring is imminent when welding the same to the other components of an ostomy faceplate.

Thus, there is still a need for an ostomy convex pressure rings combining low weight with flexibility and, at the same time being sufficiently stiff to provide and hold the convex shape of an adhesive wafer of an ostomy appliance.

The present invention provides a solution to all these problems at the same time without having to compromise with respect to one feature in order to obtain the others.

### SUMMARY OF THE INVENTION

The present Invention relates to a convex pressure ring with a radial extent between an inner periphery and an outer periphery and having a proximal side having a convex face for use together with ostomy appliance.

The present invention also relates to a body side member for use together with ostomy collecting bag and comprising a convex pressure ring with a radial extent between an inner periphery and an outer periphery and having a proximal side having a convex face.

Still further, the invention relates to an ostomy appliance in the form of a collecting bag comprising an adhesive wafer wafer for securing the appliance to the user's skin, said wafer having a hole for receiving a stoma.

### Detailed Description of the Present Invention

The present invention relates to a convex pressure ring having a proximal side having a convex face for use together with an ostomy appliance which convex pressure ring is made from light and flexible material wherein the convex pressure ring is made from
a) a solid thermoplastic elastomer such as a polyolefin elastomer in the form of a copolymer of ethylene and octene, or
b) a foamed thermoplastic elastomer in the form of a moulded foam product or an integral foam.

It has surprisingly been found that the convex pressure ring itself may be made from a flexible material combining low weight with flexibility and, at the same time a sufficient stiffness to provide and hold the convex shape of an adhesive wafer of an ostomy appliance.

When trying to adapt the softness of a convex pressure ring according to the invention this may be ensured by selecting the properties of materials as opposed to the only manner feasible in connection with corresponding known products in which only the use of a thinner material may provide a sufficient softness which requires special care with respect to design as a thin material is more in danger of damage during processing and inevitably is more susceptible to permanent deformation (due to overload) in use.

When using the above-mentioned conventional materials, a compromise must be made between obtaining flexibility and ensuring shape-stability of the convex ring.

By using a light and flexible material in accordance with the present, invention, a significantly higher flexibility may be obtained improving the wearing comfort for the user when moving and the conservation of the elastic properties of the material ensures that the convex pressure ring will resume its convex shape when the stress from moving ceases. Thus, according to the invention a convex pressure ring having a greater sectional area and thus greater strength may be provided ensuring a high load bearing ability and still cbnserving the flexibility.

It has been found that solid thermoplastic elastomers such as polyolefin elastomers in the form of copolymers of ethylene and octene or a foamed thermoplastic elastomer material in the form of a moulded foam product or an integral foam may be used for this purpose. A preferred such elastomer is Engage® 8401 Polyolefin Elastomer from DuPont Dow. Such copolymers may be Injection moulded and compression moulded, has a low density reducing the weight of the product and show a suitable stiffness for the purpose of the present invention.

Suitable such copolymers preferably have a Mooney Viscosity ML 1+4 at 121 °C as determined according to ASTM D-1646 <5 and an ultimate Tensile strength (MPa) as determined according to ASTM D-638M, 50 mm/min between 3 and 13.

Suitable foam materials may be a PE foam or an EVA/PE foam or foams made from materials selected from above-mentioned thermoplastic elastomers. Convex pressure rings according to the invention made form thermoplastic elastomer materials may be used in the existing production lines as they may be processed in an analogous manner as the conventional convex rings, only an adaptation of welding conditions which is easily determined by the skilled in the art may have to be carried out.

The convex pressure ring according to the invention may e.g. be of the type disclosed in WO 93/04648 or of the type disclosed in WO 93/18725.

In a second aspect, the invention relates to an ostomy body side member comprising an adhesive wafer wafer for securing the appliance to the user's skin, said wafer having a hole for receiving a stoma, and coupling means for exchangeable attachment of a receiving bag for receiving secretions from the ostomy, characterised in that it is provided with a convex pressure ring having a proximal side having a convex face which convex pressure ring is made from light and flexible material wherein the convex pressure ring is made from
a) a solid thermoplastic elastomer such as a polyolefin elastomer in the form of a copolymer of ethylene and octene, or
b) a foamed thermoplastic elastomer in the form of a moulded foam product or an integral foam.

An ostomy body side member according to the invention may be produced from standard materials normally used for preparation of disposable ostomy and wound and incontinence devices. Thus, the adhesive wafer may be made from a medical grade barrier adhesives known in the such as the formulation being disclosed, for example in US Patents Nos. 4,367,732, 5,051,259 or 5,714,225.

The coupling means may suitably be matching coupling rings of the type disclosed in WO 93/18725 or WO 94/18919 or matching flanges for adhesive connection of the type disclosed in U.S. Patent No. 5,800,415.

In a third aspect, the invention relates to an ostomy appliance in the form of a collecting bag comprising an adhesive wafer wafer for securing the appliance to the user's skin, said wafer having a hole for receiving a stoma and comprising a convex pressure ring with a radial extent between an inner periphery and an outer periphery, characterised in that it is provided with a convex pressure ring having a proximal side having a convex face which convex pressure ring is made from light and flexible material wherein the convex pressure ring is made from
a) a solid thermoplastic elastomer such as a polyolefin elastomer in the form of a copolymer of ethylene and octene, or
b) a foamed thermoplastic elastomer in the form of a moulded foam product or an integral foam.

A collecting bag according to the invention may be produced from standard materials normally used for preparation of disposable ostomy and wound and incontinence devices.

## Claims

1. A convex pressure ring having a proximal side having a convex face for use together with ostomy appliance which convex pressure ring is made from light and flexible material, **characterised in that** the convex pressure ring is made from
a) a solid thermoplastic elastomer such as a polyolefin elastomer in the form of a copolymer of ethylene and octene, or
b) a foamed thermoplastic elastomer in the form of a moulded foam product or an integral foam.

2. A convex pressure ring as claimed in claim 1, **characterised in that** the ring is made from a material having a Mooney Viscosity ML 1+4 at 121 °C as determined according to ASTM D-1646 <5 and an ultimate Tensile strength (MPa) as determined according to ASTM D-638M, 50 mm/min between 3 and 13 and a hardness between 70 and 100 (Shore A, ASTM-2240).

3. An ostomy body side member comprising an adhesive wafer for securing the appliance to the user's skin, said wafer having a hole for receiving a stoma, and coupling means for exchangeable attachment of a receiving bag for receiving secretions from the ostomy, **characterised in that** it is provided with a convex pressure ring having a proximal side having a convex face which convex pressure ring is made from light and flexible material wherein the convex pressure ring is made from
a) a solid thermoplastic elastomer such as a polyolefin elastomer in the form of a copolymer of ethylene and octene, or
b) a foamed thermoplastic elastomer in the form of a moulded foam product or an integral foam.

4. An ostomy appliance in the form of a collecting bag comprising an adhesive wafer for securing the appliance to the user's skin, said wafer having a hole for receiving a stoma and comprising a convex pressure ring with a radial extent between an inner periphery and an outer periphery, **characterised in that** it is provided with a convex pressure ring having a proximal side having a convex face which convex pressure ring is made from light and flexible material wherein the convex pressure ring is made from
a) a solid thermoplastic elastomer such as a polyolefin elastomer in the form of a copolymer of ethylene and octene, or
b) a foamed thermoplastic elastomer in the form of a moulded foam product or an integral foam.

## Patentansprüche

1. Konvexer Druckring mit einer proximalen Seite, die eine konvexe Außenfläche aufweist, zur Verwendung zusammen mit einer Stomavorrichtung, wobei der konvexe Druckring aus einem leichten und flexiblen Material besteht, **dadurch gekennzeichnet, dass** der konvexe Druckring hergestellt ist aus
a) einem festen thermoplastischen Elastomer wie einem Polyolefin-Elastomer in Form eines Ethylen-Octan-Copolymers oder
b) einem geschäumten thermoplastischen Elastomer in Form eines geformten Schaumprodukts oder eines Integralschaums.

2. Konvexer Druckring nach Anspruch 1, **dadurch gekennzeichnet, dass** der Ring aus einem Material hergestellt ist, das eine Mooney-Viskosität ML 1+4 bei 121 °C <5, bestimmt nach ASTM D-1646, eine Reißfestigkeit (MPa) von 3 bis 13, bestimmt nach ASTM
D-638M bei 50 mm/min, und eine Härte von 70 bis 100 (Shore A, ASTM-2240) aufweist.

3. Körperseitiges Stoma-Element, das ein Kleberplättchen zur Befestigung der Vorrichtung auf der Haut des Anwenders, das ein Loch zur Aufnahme eines Stomas besitzt, und eine Kupplungseinrichtung zur austauschbaren Anbringung eines Aufnahmebeutels zur Aufnahme von Ausscheidungen aus dem Stoma aufweist, **dadurch gekennzeichnet, dass** es mit einem konvexen Druckring ausgerüstet ist, der eine proximale Seite besitzt, die eine konvexe Außenfläche aufweist, wobei der konvexe Druckring aus einem leichten und flexiblen Material besteht und wobei der konvexe Druckring hergestellt ist aus
a) einem festen thermoplastischen Elastomer wie einem Polyolefin-Elastomer in Form eines Ethylen-Octan-Copolymers oder
b) einem geschäumten thermoplastischen Elastomer in Form eines geformten Schaumprodukts oder eines Integralschaums.

4. Stomavorrichtung in Form eines Sammelbeutels, die ein Kleberplättchen zur Befestigung der Vorrichtung auf der Haut des Anwenders aufweist, das ein Loch zur Aufnahme eines Stomas besitzt und einen konvexen Druckring mit einer radialen Ausdehnung zwischen einem inneren Umfang und einem äußeren Umfang aufweist, **dadurch gekennzeichnet, dass** sie mit einem konvexen Druckring ausgerüstet ist, der eine proximale Seite besitzt, die eine konvexe Außenfläche aufweist, wobei der konvexe Druckring aus einem leichten und flexiblen Material besteht und wobei der konvexe Druckring hergestellt ist aus
a) einem festen thermoplastischen Elastomer wie einem Polyolefin-Elastomer in Form eines Ethylen-Octan-Copolymers oder
b) einem geschäumten thermoplastischen Elastomer in Form eines geformten Schaumprodukts oder eines Integralschaums.

## Revendications

1. Anneau de pression convexe ayant un côté proximal doté d'une face convexe pour l'utilisation conjointe avec un dispositif pour stomie, ledit anneau de pression convexe étant fait en un matériau léger et flexible, **caractérisé en ce que** l'anneau de pression convexe est fabriqué à partir de :
a) un élastomère thermoplastique solide comme un élastomère polyoléfinique, sous forme de copolymère d'éthylène et d'octène, ou
b) un élastomère thermoplastique foisonné sous la forme d'un produit de mousse moulé ou d'une mousse intégrale.

2. Anneau de pression convexe selon la revendication 1, **caractérisé en ce que** l'anneau est fabriqué à partir d'un matériau ayant une viscosité de Mooney ML 1+4 à 121°C, déterminée selon la norme ASTM D-1646, < 5 et une résistance à la traction à rupture (MPa), déterminée selon la norme ASTM D-638M, à 50 mm/min, entre 3 et 13 et une dureté entre 70 et 100 (Shore A, ASTM-2240).

3. Eiément côté corps pour stomie, comprenant une gaze adhésive pour fixer le dispositif sur la peau de l'utilisateur, ladite gaze ayant un orifice pour recevoir la stomie, et un moyen de couplage pour la fixation amovible d'une poche collectrice destinée à recevoir des sécrétions provenant de la stomie, **caractérisé en ce qu'**il est muni d'un anneau de pression convexe ayant un côté proximal doté d'une face convexe, ledit anneau de pression convexe est fait en un matériau léger et flexible, l'anneau de pression convexe étant fabriqué à partir de :
a) un élastomère thermoplastique solide comme un élastomère polyoléfinique, sous forme de copolymère d'éthylène et d'octène, ou
b) un élastomère thermoplastique foisonné sous la forme d'un produit de mousse moulé ou d'une mousse intégrale.

4. Dispositif pour stomie prenant la forme d'une poche collectrice, comprenant une gaze adhésive pour fixer le dispositif à la peau de l'utilisateur, ladite gaze ayant un orifice pour recevoir une stomie et comprenant un anneau de pression convexe doté d'une extension radiale entre une périphérie interne et une périphérie externe, **caractérisé en ce qu'**il est muni d'un anneau de pression convexe ayant un côté proximal doté d'une face convexe, ledit anneau de pression convexe est fait en un matériau léger et flexible, l'anneau de pression convexe étant fabriqué à partir de :
a) un élastomère thermoplastique solide comme un élastomère polyoléfinique, sous forme de copolymère d'éthylène et d'octène, ou
b) un élastomère thermoplastique foisonné sous la forme d'un produit de mousse moulé ou d'une mousse intégrale.
